# NOUVEAU FASCICULE DE BREVET EUROPEEN

(11) **EP 3 151 758 B2**
(45) Date de publication et mention de la décision concernant l'opposition: **12.03.2025**
(45) Mention de la délivrance du brevet: 08.06.2022
(21) Numéro de dépôt: 15725711.4
(22) Date de dépôt: 31.03.2015
(51) Int. Cl.: A61B 17/12, A61B 5/00, A61M 5/44, A61M 5/42, A61B 5/15, A61B 5/153, A61B 17/132

(54) **DISPOSITIF DE MAINTIEN D'UNE VEINE D'UN UTILISATEUR EN POSITION ET DISPOSITIF DE PONCTION OU D'INJECTION DANS UNE VEINE D'UN UTILISATEUR**
VORRICHTUNG ZUR AUFRECHTERHALTUNG DER VENE EINES BENUTZERS IN EINER POSITION UND VORRICHTUNG ZUR PUNKTIERUNG ODER EINSPRITZUNG IN DIE VENE EINES BENUTZERS
DEVICE FOR MAINTAINING A USER'S VEIN IN POSITION AND DEVICE FOR PUNCTURING OR INJECTING INTO A USER'S VEIN

(30) Priorité: 14.04.2014 FR 1400893
(43) Date de publication de la demande: 12.04.2017
(73) Titulaire: BHEALTHCARE, 44800 Saint-Herblain (FR)
(72) Inventeur: BRETEAU, Aliaume, F-75725 Paris (FR); BALP, Adrien, F-75725 Paris (FR); GUYOT, Pauline, F-75725 Paris (FR); NAVARRO, Arnaud, F-75725 Paris (FR); SABOURET, Maxime, F-75725 Paris (FR)
(74) Mandataire: Bringer, Mathieu
(86) Numéro de dépôt international: PCT/FR2015/050842
(87) Numéro de publication internationale: WO 2015/158978

(56) Documents cités:
- EP-A1- 2 289 578
- DE-A1- 102005 028 263
- DE-A1- 4 115 515
- JP-A- 2003 310 578
- US-A- 2 525 398
- US-A- 3 324 854
- US-A1- 2006 129 184
- US-A1- 2010 274 202
- US-A1- 2010 274 202
- US-A1- 2012 190 981
- US-A1- 2012 190 981
- US-A1- 2013 041 253
- US-B1- 6 398 726
- US-B1- 8 888 714

## Description

### DOMAINE TECHNIQUE DE L'INVENTION

La présente invention vise un dispositif de maintien d'une veine d'un utilisateur en position et un dispositif de ponction ou d'injection dans une veine d'un utilisateur. Elle s'applique, notamment, aux ponctions sanguines, aux injections sanguines et à la pose de cathéters.

### ETAT DE LA TECHNIQUE

Dans le milieu hospitalier, les prises de sang ainsi que les poses de cathéter sont des procédures très répétitives et extrêmement chronophages pour le personnel médical. Mobilisant une grande proportion de membres du corps médical, les erreurs et blessures dues à ce type d'intervention sont fréquentes et représentent une charge supplémentaire d'ordre financier à l'hôpital.

En effet, tous les jours, des millions de piqûres sont effectues à l'échelle mondiale. Chaque année, on reporte plusieurs millions de blessures causées par des piqûres mal engagées et il est estimé, de plus, que 55% des erreurs ne sont pas reportées. De nombreux praticiens se blessent également avec les aiguilles et sont infectés par les germes du patient entraînant dans certains cas de graves complications médicales.

Par ailleurs, le risque pour un professionnel de la santé d'être contaminé par un patient atteint d'une maladie comme le SIDA (virus VIH) par exemple, renforce le danger lié à la prise en charge de ces patients.

De plus, les systèmes actuels ne permettent pas de diminuer la peur d'un patient pour une aiguille ni la douleur provoquée par l'insertion cutanée de cette aiguille. DE 10 2005 028263 divulgue un dispositif de détection d'une veine superficielle mettant en œuvre une diode électroluminescente configurée pour émettre des rayons lumineux , cependant ce document ne décrit pas un moyen de capture d'une image infrarouge du bras de L'utilisateur. US 2012/190981 divulgue un système d'insertion par voie intraveineuse comprenant un bras de robot sur lequel sont fixés un ensemble de capteur, un outil de maintien d'une aiguille et des pieds de stabilisation.

### OBJET DE L'INVENTION

La présente invention vise à remédier à tout ou partie de ces inconvénients.

A cet effet, selon un premier aspect, la présente invention vise un dispositif de maintien d'une veine d'un utilisateur en position, qui comporte :
- au moins deux branches séparées par un interstice d'une largeur supérieure à la dimension de la veine et
- un moyen de positionnement des branches autour de la veine.

Grâce à ces dispositions, une veine à laquelle un traitement médical doit être réalisé est maintenue en position par les branches du dispositif.

Dans des modes de réalisation, le dispositif objet de la présente invention comporte :
- un moyen d'échauffement d'au moins une branche et
- un moyen de commande du moyen d'échauffement.

Ces modes de réalisation ont l'avantage de permettre une dilatation de la veine à laquelle un traitement droit être réalisé.

Dans des modes de réalisation, le dispositif objet de la présente invention comporte un moyen de refroidissement d'au moins une branche, le moyen de commande étant configuré pour actionner le moyen de refroidissement après l'actionnement du moyen d'échauffement.

L'avantage de ces modes de réalisation est qu'ils permettent, par exemple grâce à l'utilisation d'un effet Peltier, d'anesthésier une zone du corps de l'utilisateur autour de la veine à laquelle un traitement doit être réalisé. La sensation de froid traversant le système nerveux plus rapidement que la sensation de douleur, l'utilisateur ressent le froid en lieu de la sensation de douleur.

Dans des modes de réalisation, le dispositif objet de la présente invention comporte un moyen d'émission de lumière ultraviolette en direction de la veine de l'utilisateur.

Ces modes de réalisation ont l'avantage de stériliser la peau autour de la veine ainsi que tout équipement médical autour de cette zone.

Selon l'invention, le dispositif objet de la présente invention comporte en outre un moyen de désinfection de la peau couvrant la veine de l'utilisateur.

L'avantage est qu'il permet d'éviter à l'utilisateur de risquer une infection au cours d'une procédure médicale réalisée sur la veine à traiter.

Dans des modes de réalisation, le moyen de positionnement comporte un moyen de pincemnet de la veine par les branches.

Ces modes de réalisation ont l'avantage de permettre de maintenir la veine en position et d'augmenter la surface de contact entre les branches et la peau de l'utilisateur.

Selon l'invention, le dispositif de ponction ou d'injection dans une veine d'un utilisateur, comporte en outre :
- un dispositif de maintien d'une veine d'un utilisateur en position et
- un boîtier opaque comportant une ouverture pour recevoir un bras de l'utilisateur.

Grâce à ces dispositions, la vue d'un traitement réalisé à la veine de l'utilisateur est cachée à cet utilisateur de manière à éviter à l'utilisateur de ressentir la peur, par exemple.

Selon l'invention, le dispositif de ponction ou d'injection objet de la présente invention comporte :
- un moyen de capture d'image infrarouge du bras reçu dans le boîtier,
- un moyen de détection d'une veine dans l'image détectée,
- un moyen de transmission d'une localisation de la veine détectée,
- le moyen de positionnement des branches autour de la veine étant commandé en fonction de la localisation de la veine.

L'avantage est qu'il permet de réaliser automatiquement le positionnement des branches.

Selon l'invention, le dispositif de ponction ou d'injection objet de la présente invention comporte :
- une aiguille
- un moyen de positionnement d'une extrémité de la veine détectée, entre les branches.

Ainsi, l'invention permet l'insertion de l'aiguille dans la veine détectée de manière à réaliser un traitement sur la veine.

Dans des modes de réalisation, le dispositif de ponction ou d'injection objet de la présente invention comporte :
- un moyen d'émission d'ultrason et
- un moyen de capture d'une image en fonction des ultrasons émis,
le moyen de positionnement positionnant l'aiguille en fonction de l'image captée.

Ces modes de réalisation ont l'avantage de ne pas être sensible à une émission de chaleur d'une branche pouvant interférer dans la détection de la veine.

Dans des modes de réalisation, le dispositif de ponction ou d'injection objet de la présente invention comporte un moyen de permutation entre une aiguille ayant réalisé une ponction et/ou une injection et une autre aiguille.

L'avantage de ces modes de réalisation est qu'ils permettent d'automatiser le changement d'aiguille se produisant entre deux traitements réalisés par le dispositif.

Dans des modes de réalisation, le dispositif de ponction ou d'injection objet de la présente invention comporte un moyen de maintien du bras de l'utilisateur dans le boîtier.

Ces modes de réalisation ont l'avantage de diminuer les risques de blessure pouvant résulter d'un retrait imprévu du bras de l'utilisateur.

Dans des modes de réalisation, le moyen de maintien comporte un garrot se contractant automatiquement autour du bras de l'utilisateur et une poignée pour recevoir la main fermée de l'utilisateur.

L'avantage de ces modes de réalisation est de permettre un maintien du bras à deux points dont la stabilité est améliorée.

Dans des modes de réalisation, le dispositif de ponction ou d'injection objet de la présente invention comporte :
- un moyen d'aspiration de sang issu de la veine dans lequel l'aiguille est positionnée et
- un réservoir amovible pour recevoir le sang aspiré.

Ces modes de réalisation ont l'avantage de permettre d'automatiser un traitement de type ponction réalisé à la veine.

Dans des modes de réalisation, le dispositif de ponction ou d'injection objet de la présente invention comporte :
- un moyen d'accès à un profil de l'utilisateur,
- un moyen d'identification du réservoir avec une donnée du profil de l'utilisateur.

L'avantage de ces modes de réalisation est de permettre d'identifier un réservoir en fonction de la donnée du profil d'utilisateur.

### BREVE DESCRIPTION DES FIGURES

D'autres avantages, buts et caractéristiques particulières de l'invention ressortiront de la description non limitative qui suit d'au moins un mode de réalisation particulier du dispositif de maintien d'une veine d'un utilisateur en position et du dispositif de ponction ou d'injection dans une veine d'un utilisateur objets de la présente invention, en regard des dessins annexés, dans lesquels :
- la figure 1 représente, schématiquement et en coupe, un mode de réalisation particulier du dispositif de maintien objet de la présente invention,
- la figure 2 représente, schématiquement et en coupe, un premier mode de réalisation particulier du dispositif de ponction ou d'injection objet de la présente invention,
- la figure 3 représente, schématiquement et en coupe, un logigramme d'étapes particulier du procédé objet de la présente invention et
- la figure 4 représente, schématiquement et en coupe, un deuxième mode de réalisation particulier du dispositif de ponction ou d'injection objet de la présente invention,
- la figure 5 représente, schématiquement et en coupe, un troisième mode de réalisation particulier du dispositif de ponction ou d'injection objet de la présente invention et
- la figure 6 représente, schématiquement et en coupe, un quatrième mode de réalisation particulier du dispositif de ponction ou d'injection objet de la présente invention.

### DESCRIPTION D'EXEMPLES DE REALISATION DE L'INVENTION

La présente description est donnée à titre non limitatif, chaque caractéristique d'un mode de réalisation pouvant être combinée à toute autre caractéristique de tout autre mode de réalisation de manière avantageuse. Par ailleurs, chaque paramètre d'un exemple de réalisation peut être mis en oeuvre indépendamment d'autres paramètres dudit exemple de réalisation.

On note que le terme « un » est utilisé au sens de « au moins un ».

On note dès à présent que les figures ne sont pas à l'échelle.

On note que les traitements informatiques réalisés sont, par exemple, réalisés par un mini-ordinateur de type Raspberry Pi (marque déposée).

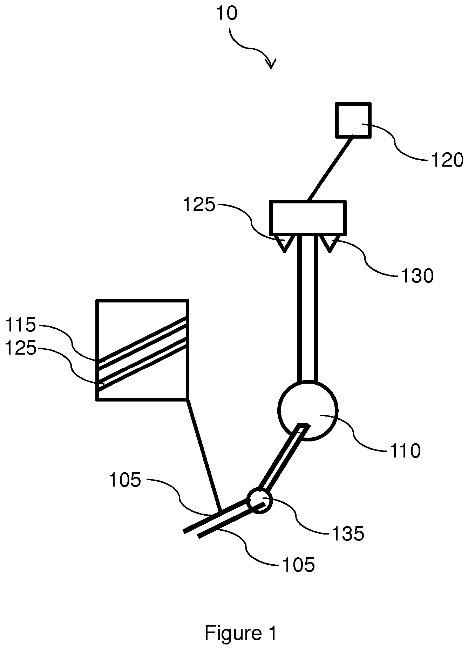

On observe, sur la figure 1, qui n'est pas à l'échelle, une vue en coupe d'un mode de réalisation du dispositif 10 de maintien d'une veine d'un utilisateur en position objet de la présente invention. Ce dispositif 10 comporte :
- au moins deux branches 105 séparées par un interstice d'une largeur supérieure à la dimension de la veine et
- un moyen 110 de positionnement des branches 105 autour de la veine comportant un moyen 135 de pincement de la veine par les branches 105,
- un moyen 115 d'échauffement d'au moins une branche 105,
- un moyen 125 de refroidissement d'au moins une branche 105,
- un moyen 120 de commande du moyen 115 d'échauffement et du moyen 125 de refroidissement,
- un moyen 125 d'émission de lumière ultraviolette en direction de la veine de l'utilisateur et
- un moyen 130 de désinfection de la peau couvrant la veine de l'utilisateur.

Les deux branches 105 sont, par exemple, des lamelles de métal parallèles séparées par un interstice d'une largeur d'un centimètre. Ces lamelles peuvent être en tout type de matériau rigide, tel du plastique, de la céramique ou du verre par exemple.

Dans des variantes préférentielles, chaque branche 105 est configurée pour réaliser un effet Peltier. Ces branches 105 comportent alors deux faces, en céramique électroisolante par exemple, séparées par des semi-conducteurs agissant comme contacts entre deux conducteurs électriques positionnés chacun au niveau d'une face. Dans des variantes, certains semi-conducteurs sont dopés de type P et d'autres semi-conducteurs sont dopés de type N. L'application d'un courant dans l'un des conducteurs électriques entraîne une émission de chaleur par l'une des faces et une absorption de chaleur par l'autre face. Dans ces variantes, tout type de cellule Peltier existante et connue de l'Homme du Métier peut être utilisée.

Ces branches 105 sont positionnées par le moyen de positionnement 110. Ce moyen de positionnement 110 est, par exemple, un bras configuré pour déplacer en translation selon trois axes un moyen de rotation des branches 105. Ce moyen de rotation des branches 105 comporte un moyen de verrouillage de position de manière à ce que les branches 105 soient contraintes en mouvement une fois le positionnement effectué.

Ce moyen de rotation des branches 105 est associé à un moyen de pincement 135 de la veine par les branches 105. Ce moyen de pincement 135 est, par exemple, un moteur configuré pour rapprocher les branches 105 l'une de l'autre de manière à pincer la peau d'un utilisateur. Le moyen de positionnement 110 peut être motorisé ou non.

Dans des variantes, le moyen de positionnement 110 est monté sur un pied et articulé de sorte à ce qu'un utilisateur positionne le moyen de positionnement 110 par déplacement dans l'espace d'une tête comportant les branches 105.

Le moyen d'échauffement 115 est, par exemple, un ensemble formé par deux faces d'orientation similaire des branches 105. Ces deux faces sont, par exemple, des faces similaires de cellules Peltier telles que décrites ci-dessus. Ces deux faces sont configurées pour émettre conjointement ou indépendamment de la chaleur.

Le moyen de refroidissement 125 est, par exemple, un ensemble formé par les deux autres faces des branches 105. Ces deux autres faces ont des orientations similaires. Ces deux faces sont, par exemple, des faces similaires de cellules Peltier telles que décrites ci-dessus. Ces deux faces sont configurées pour absorber conjointement ou indépendamment de la chaleur.

Dans des variantes, le dispositif 10 comporte un moyen de rotation des branches 105 de manière à échauffer d'abord une partie de la peau d'un utilisateur autour de la veine à traiter puis à refroidir la même partie.

L'actionnement du moyen d'échauffement 115 et du moyen de refroidissement 125 est commandé par le moyen de commande 120. Ce moyen de commande 120 est, par exemple, un circuit imprimé comportant un microcontrôleur. Ce microcontrôleur est configuré pour commander la transmission d'un courant électrique à chaque cellule Peltier.

Le moyen 120 de commande est configuré pour actionner le moyen de refroidissement 125 après l'actionnement du moyen d'échauffement 115.

Dans des variantes, le moyen de commande 120 est commandé par l'actionnement d'un bouton du dispositif 10 par un utilisateur.

Le moyen d'émission 125 d'une lumière ultraviolette est, par exemple, une ou une pluralité de lampes ultraviolettes. Chaque lampe est configurée pour être actionnée par un microcontrôleur du circuit imprimé. L'actionnement de chaque lampe peut être automatique et réalisé lorsque le moyen de positionnement 110 est verrouillé.

Dans des variantes, chaque lampe est actionnée par l'actionnement d'un bouton du dispositif 10 par un utilisateur.

Le moyen de désinfection 130 est, par exemple, un pulvérisateur d'un liquide de désinfection. Dans des variantes, ce moyen de désinfection 130 est une brosse connectée à un bras motorisé configuré pour réaliser un frottement du bras de l'utilisateur par la brosse. Ce bras motorisé est actionné, par exemple, par un microcontrôleur du circuit imprimé.

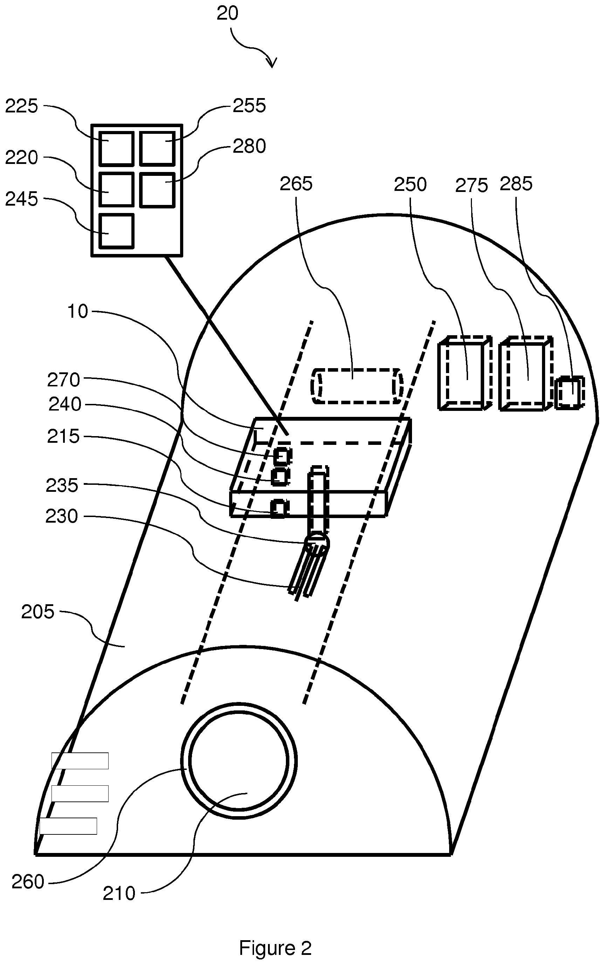

On observe, sur la figure 2, qui n'est pas à l'échelle, une vue d'un premier mode de réalisation du dispositif 20 de ponction ou d'injection dans une veine d'un utilisateur objet de la présente invention. Ce dispositif 20 comporte :
- un dispositif 10 de maintien d'une veine d'un utilisateur en position,
- un boîtier 205 opaque comportant une ouverture 210 pour recevoir un bras de l'utilisateur,
- un moyen 215 de capture d'image infrarouge du bras reçu dans le boîtier 205,
- un moyen 220 de détection d'une veine dans l'image détectée,
- un moyen 225 de transmission d'une localisation de la veine détectée,
- le moyen 110 de positionnement des branches 105 autour de la veine étant commandé en fonction de la localisation de la veine,
- une aiguille 230,
- un moyen 235 de positionnement d'une extrémité de l'aiguille dans la veine détectée, entre les branches 105,
- un moyen 240 d'émission d'ultrason,
- un moyen 245 de capture d'une image en fonction des ultrasons émis,
- un moyen 250 de permutation entre une aiguille 230 ayant réalisé une ponction et/ou une injection et une autre aiguille,
- un moyen 255 de maintien du bras de l'utilisateur dans le boîtier qui comporte :
   - un garrot 260 se contractant automatiquement autour du bras de l'utilisateur et
   - une poignée 265 pour recevoir la main fermée de l'utilisateur ;
- un moyen 270 d'aspiration de sang issu de la veine dans lequel l'aiguille 230 est positionnée,
- un réservoir 275 amovible pour recevoir le sang aspiré.
- un moyen 280 d'accès à un profil de l'utilisateur et
- un moyen 285 d'identification du réservoir 275 avec une donnée du profil de l'utilisateur.

Le dispositif 10 est similaire au dispositif 10 décrit en figure 1. Ce dispositif 10 est positionné dans un boîtier 205 opaque. Ce boîtier 205 est, par exemple, une structure en forme de demi cylindre de rotation comportant sur l'une des faces planes une ouverture 210. Cette ouverture 210 est configurée pour recevoir un bras d'un utilisateur et peut être de toute forme géométrique.

Afin de mettre en mouvement le moyen de positionnement 110 du dispositif 10, le dispositif 10 est fixé à deux rails traversant longitudinalement le boîtier 205. Ces rails comportent chacun une crémaillère sur le long de laquelle le dispositif 10 se déplace. Une seconde crémaillère, orientée selon un axe orthogonal aux deux rails longitudinaux, permet de positionner le dispositif 10 selon un axe transversal du boîtier 205. Une troisième crémaillère orientée selon un axe vertical et orthogonal aux deux autres axes des première et deuxième crémaillères permet de positionner le dispositif 10 en hauteur. Le positionnement du dispositif 10 peut être réalisé grâce à l'utilisation d'un ensemble de moteurs pas à pas.

Dans des variantes, le positionnement du dispositif 10 est réalisé par un bras robotisé comportant deux articulations.

Le moyen de capture 215 d'image infrarouge du bras reçu dans le boîtier 205 est, par exemple, une caméra infrarouge configurée pour capter une image dans le spectre infrarouge. Ce moyen de capture 215 est positionné sur le dispositif 10 mobile et orienté vers le bras de l'utilisateur. Dans des variantes, le moyen de capture 215 est positionné sur un module se déplaçant le long des crémaillères longitudinales. Dans ces variantes le dispositif 10 peut être immobile le long desdites crémaillères et positionné au bout des rails longitudinaux.

Dans des variantes, le moyen de capture 215 est associé à un polariseur.

Le moyen de détection 220 d'une veine dans l'image détectée est, par exemple, un programme informatique embarqué dans un composant du circuit imprimé du dispositif 10. Ce composant est connecté au moyen de capture 215 de sorte à ce que l'image captée soit transférée au moyen de détection 220. Ce programme informatique détermine par traitement d'image, par exemple par détection de contours, le positionnement des veines le long du bras de l'utilisateur. Ce moyen de détection 220 sélectionne une veine parmi les veines détectées en fonction de la largeur de la veine détectée. Préférentiellement, la veine la plus large est sélectionnée.

Dans des variantes, le programme informatique met en œuvre une conversion en niveau de gris, une augmentation du contraste, un seuillage adaptatif puis une détection des contours.

Dans des variantes, une pluralité de diodes électroluminescentes, organisées en couronne, illumine le bras.

Le moyen d'émission 240 d'ultrason est, par exemple, un émetteur à ultrason positionné à proximité du dispositif 10 et configuré pour émettre des ultrasons en direction du bras de l'utilisateur. Ce moyen d'émission 240 reçoit une commande d'émission d'ultrason émise par un composant du circuit électronique du dispositif 10. Cette commande est émise une fois qu'une veine a été détectée par le moyen de détection 220.

Le moyen de capture 245 d'une image en fonction des ultrasons émis est, par exemple, un capteur à ultrason. Ce capteur à ultrason permet notamment de détecter la profondeur d'une veine détectée par le moyen de détection 220. Si la profondeur détectée est supérieure à une valeur limite prédéterminée, le moyen de détection 220 sélectionne une autre veine et le moyen d'émission 240 d'ultrason et de nouveau mis en œuvre.

Dans des variantes, le moyen d'émission 240 et le moyen de capture 245 sont remplacés par un moyen d'émission d'ultrasons dans l'aiguille 230 et un capteur de la fréquence de résonance de l'aiguille 230 en fonction du signal d'ultrasons émis. Dans l'air, l'aiguille présente une fréquence de résonance propre qui varie légèrement lors de l'insertion de ladite aiguille dans la peau de l'utilisateur. Lorsque l'aiguille pénètre dans une veine, la fréquence de résonance varie brutalement, ce qui indique au dispositif qu'une veine a été percée. Dans des variantes, tout type de dispositif de dispositif d'imagerie à ultrason peut être utilisé.

Dans des variantes, le dispositif 20 comporte un moyen de détection de la pénétration de l'aiguille 230 dans une veine. Ce moyen de détection est, par exemple, un cristal piézo-électrique configuré pour émettre un signal électrique en fonction d'une variation de position longitudinale de l'aiguille 230 spontanée et de faible amplitude, cette variation correspondant à la percée d'une paroi de la veine par l'aiguille 230.

Dans des variantes, le dispositif 20 comporte un moyen de détection de la profondeur d'une veine détectée. Ce moyen de détection détecte la profondeur d'une veine en faisant parcourir le bras de l'utilisateur par un capteur de relief. L'imagerie de relief, associée à l'imagerie infrarouge, permet de sélectionner la veine de l'utilisateur.

Le moyen de positionnement 110 des branches 105 autour de la veine est, par exemple, le moyen de positionnement 110 du dispositif 10 décrit en figure 1. Ce moyen de positionnement 110 est commandé par un microcontrôleur du dispositif 10. Ce microcontrôleur est configuré pour positionner le moyen de positionnement 110 en fonction de la localisation de la veine détectée. Le dispositif 10 est positionné de sorte que les branches 105 pincent la peau au niveau de la veine détectée.

Dans des variantes, le dispositif 20 comporte un moyen de vérification du maintien de la veine. Ce moyen de vérification est un composant du circuit électronique du dispositif 10 configuré pour commander au moyen de capture 215 et au moyen de détection 220 de réaliser une nouvelle détection de la veine. Si la veine détectée n'est pas maintenue, le positionnement des branches 105 est réalisé de nouveau.

Le dispositif 20 comporte une aiguille 230. Cette aiguille 230 est, par exemple, positionnée dans un support d'aiguille 230 solidaire du moyen de positionnement 110. Cette aiguille 230 est, dans des variantes, parallèle aux branches 105 et initialement rétractée entre les branches. Lorsqu'une insertion de l'aiguille 230 dans la peau de l'utilisateur est réalisée, l'aiguille 230 est poussée de manière à dépasser du plan formé par les deux branches 105.

Cette insertion de l'aiguille 230 est réalisée par le moyen de positionnement 235. Ce moyen de positionnement 235 est, par exemple, un moteur configuré pour déplacer en translation l'aiguille 230.

Dans des variantes, l'aiguille 230 est configurée pour réaliser une ponction, une injection ou une pose de cathéter.

Le moyen 250 de permutation entre une aiguille 230 ayant réalisé une ponction et/ou une injection et une autre aiguille est, par exemple, un distributeur d'aiguilles. Ce distributeur comporte un réservoir à aiguilles stériles et une cavité permettant le passage d'une seule aiguille à la fois. Chaque aiguille s'insérant dans la cavité est maintenue par un support d'aiguille. Lorsqu'une aiguille utilisée est relâchée par le dispositif 10 dans un réservoir à aiguilles usagées, un support d'aiguille est assemblé par vissage dans le dispositif 10 de manière à remplacer le support d'aiguille précédent. Ce moyen de permutation 250 est positionné, par exemple, à une extrémité du boîtier 205 opposée à l'ouverture 210.

Le moyen de maintien 255 du bras de l'utilisateur dans le boîtier 205 est, par exemple, un composant du circuit électronique du dispositif 10 configuré pour actionner une contraction du garrot 260.

Ce garrot 260 est, par exemple, un garrot pneumatique entourant l'ouverture 210 est configuré pour être gonflé à la réception d'une commande émise par le moyen de maintien 255. Dans des variantes, ce garrot 260 est un fil tendu par l'actionnement d'un moteur entraînant une réduction du périmètre du fil autour du bras.

Le moyen de maintien 255 comporte, de plus, une poignée 265 pour recevoir la main fermée de l'utilisateur. Cette poignée 265 est positionnée, par exemple, à une extrémité du boîtier 205 opposée par rapport à l'ouverture 210.

Le moyen 270 d'aspiration de sang issu de la veine dans lequel l'aiguille 230 est positionnée est, par exemple, une pompe configurée pour aspirer du sang depuis la veine jusqu'à un réservoir 275 amovible pour recevoir le sang aspiré. Ce réservoir 275 est, par exemple, une poche en matière plastique.

Le moyen 280 d'accès à un profil de l'utilisateur est, par exemple, une carte réseau configurée pour se connecter à un réseau de données type internet ou intranet et pour, en fonction d'un identifiant d'utilisateur entré par un utilisateur, extraire des données d'utilisateur d'une base de donnée. Ces données d'utilisateur peuvent être, par exemple, un nom, prénom, âge, sexe, type sanguin.

Le moyen d'identification 285 du réservoir 275 avec une donnée du profil de l'utilisateur est, par exemple, un circuit électronique configuré pour rentrer les données extraites dans une puce NFC associée au réservoir. Dans des variantes, ce moyen d'identification 285 est une imprimante configurée pour imprimer une donnée extraite sur une étiquette autocollante collée au réservoir 275.

Le dispositif 20 comporte, dans des variantes, un bouton de sécurité configuré pour provoquer un retrait de l'aiguille 230 et une mise à l'arrêt du dispositif 20.

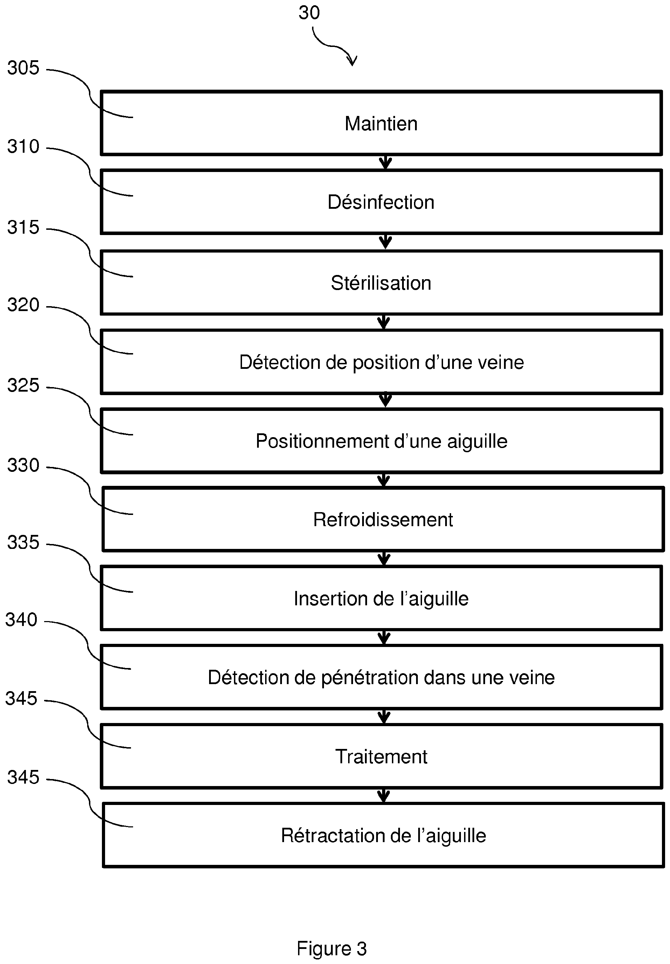

On observe, sur la figure 3, qui n'est pas à l'échelle, une vue en coupe d'un mode de réalisation du procédé 30 objet de la présente invention. Ce procédé 30 comporte :
- une étape de maintien 305 d'un bras d'un utilisateur dans le dispositif 20,
- une étape de désinfection 310 de la peau couvrant la veine,
- une étape de stérilisation 315 de la peau,
- une étape de détection 320 d'une veine,
- une étape de positionnement 325 du dispositif de maintien d'une veine,
- une étape de refroidissement 330 de la peau,
- une étape d'insertion 335 d'une aiguille dans la veine d'un utilisateur,
- une étape de détection 340 d'une profondeur de profondeur de pénétration de l'aiguille dans la veine,
- une étape de réalisation d'un traitement 345 à la veine et
- une étape de rétractation de l'aiguille 350 de la veine.

L'étape de maintien 305 est réalisée, par exemple, par la mise en oeuvre d'un garrot et d'une poignée tels que décrits en figure 2. Lorsque l'utilisateur a positionné un bras à l'intérieur du boîtier du dispositif 20 tel que décrit en figure 2 et attrapé la poignée, le garrot est actionné. Ce garrot entoure le bras de l'utilisateur au niveau de l'ouverture du boîtier.

L'étape de désinfection 310 est réalisée, par exemple, par la mise en oeuvre d'un moyen de désinfection tel que décrit en figure 2. Une fois que le garrot est actionné, le moyen de désinfection désinfecte la peau du bras de l'utilisateur.

L'étape de stérilisation 315 est réalisée, par exemple, par la mise en oeuvre d'un moyen d'émission d'une lumière ultraviolette tel que décrit en figure 1. Une fois que le moyen de désinfection a désinfecté le bras de l'utilisateur, une lumière ultraviolette est émise de manière à stériliser l'environnement intérieur du boîtier du dispositif 20.

L'étape de détection 320 est réalisée, par exemple, par la mise en œuvre du moyen de capture d'image infrarouge et du moyen de détection tels que décris en figure 2. Au cours de cette étape, une veine est détectée par détection de contours puis sélectionnée en fonction de la largeur détectée de la veine. Dans des variantes, l'utilisation d'une détection d'imagerie ultrason permet de détecter la profondeur de la veine.

Dans des variantes, un utilisateur sélectionne la veine sur un écran d'un dispositif externe connecté au dispositif réalisant le traitement. Un tel écran est monté sur une tablette numérique, par exemple.

Le dispositif de piquée communique avec le dispositif externe par le biais d'une technologie sans-fil de type Bluetooth, Wi-Fi ou Zigbee par exemple.

L'étape de positionnement 325 est réalisée, par exemple, par la mise en œuvre d'un moyen de positionnement tel que décrit en figure 1. Le moyen de positionnement positionne les branches du dispositif de manière à entourer une veine détectée de l'utilisateur.

L'étape de positionnement 325 met en œuvre, par exemple :
- le déplacement de l'aiguille vers la zone de piquée,
- le positionnement de l'aiguille au-dessus de la zone de piquée,
- la piquée de l'aiguille jusqu'à la pénétration de la veine,
- l'inclinaison de l'aiguille pour qu'elle puisse remonter dans la veine,
- une deuxième poussée de l'aiguille pour qu'elle remonte dans la veine et
- le retrait de l'aiguille.

Dans des variantes, une étape d'échauffement est réalisée, par exemple, par la mise en œuvre d'un moyen d'échauffement tel que décrit en figure 1. Ce moyen s'échauffement échauffe la peau du bras de l'utilisateur.

L'étape de refroidissement 330 est réalisée, par exemple, par un moyen de refroidissement tel que décrit en figure 1. Au cours de cette étape, le bras de l'utilisateur est refroidit par le moyen de refroidissement.

L'étape d'insertion 335 de l'aiguille est réalisée par actionnement d'un moteur pour déplacer l'aiguille dans une veine de l'utilisateur.

Dans des variantes, le procédé 30 comporte une étape de confirmation d'une insertion à réaliser, cette étape mettant en œuvre, par exemple, par une saisie de commande d'utilisateur sur un terminal portable communicant. Un tel terminal portable communicant est, par exemple, une tablette numérique. La saisie de commande met en œuvre une interface homme-machine, tel un écran tactile par exemple.

L'étape de détection 340 d'une profondeur de pénétration est réalisée, par exemple, par détection d'ultrasons émis dans l'aiguille variant en fonction de la position de l'aiguille. Dans l'air, l'aiguille présente une fréquence de résonance propre qui varie légèrement lors de l'insertion de ladite aiguille dans la peau de l'utilisateur. Lorsque l'aiguille pénètre dans une veine, la fréquence de résonance varie brutalement, ce qui indique au dispositif qu'une veine a été percée. Dans des variantes, tout type de dispositif de dispositif d'imagerie à ultrason peut être utilisé.

L'étape de réalisation d'un traitement 345 est réalisée, par exemple, par une aiguille telle que décrite en figure 2. Ce traitement peut correspondre à une ponction, une injection ou à la mise en place d'un cathéter.

L'étape de rétractation 350 est réalisée, par exemple, par mise en œuvre du moyen de positionnement tel que décrit en figure 1. Une fois le traitement réalisé, l'aiguille est retirée de la veine de l'utilisateur.

Dans des variantes, le procédé 30 comporte une étape d'enregistrement sur une base de données d'une information représentative de la nature et d'une quantité de produit injecté, d'un volume de sang prélevé ou de la pose d'un cathéter. Dans d'autres variantes, une information d'horodatage correspondant à la réalisation du traitement est enregistrée.

Les informations ainsi enregistrées peuvent être consultées avant la réalisation d'un nouveau traitement. En fonction de ces informations, par exemple, une quantité de produit et/ou la nature d'un produit à injecter est déterminée de manière automatique.

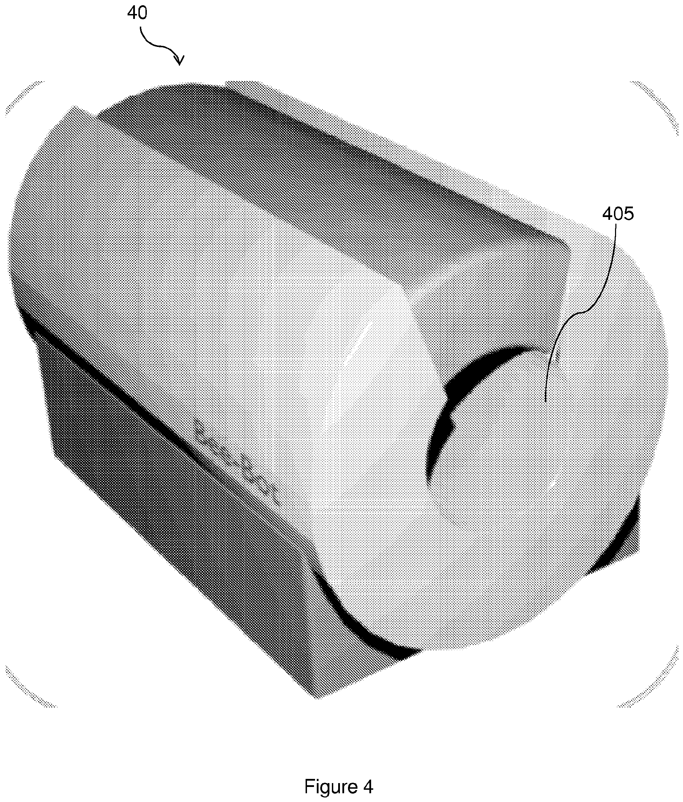

On observe sur la figure 4, schématiquement et en perspective, un deuxième mode de réalisation du dispositif 40 de ponction ou d'injection objet de la présente invention. Ce dispositif 40 présente une ouverture 405 dans un boîtier en forme grossièrement cylindrique. L'intérieur de ce boîtier est similaire au dispositif 20 objet de la présente invention.

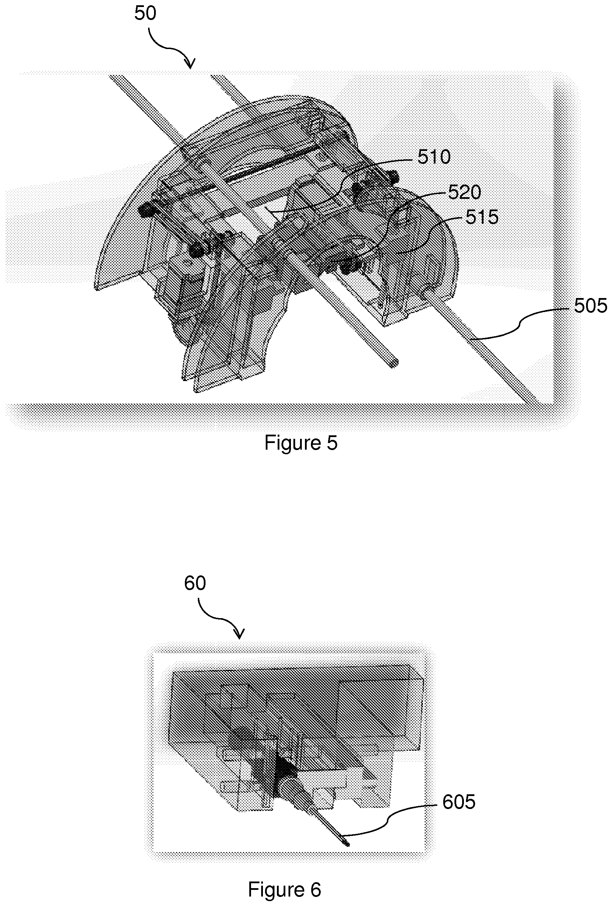

On observe sur la figure 5, schématiquement et en perspective, un troisième mode de réalisation du dispositif 50 de ponction ou d'injection objet de la présente invention. Sur cette figure 5, on observe notamment un rail 505 de déplacement d'un module dit « de piquée », c'est à dire comportant une aiguille 510. Ce module de piquée comporte un plateau 515 se déplaçant en altitude, c'est à dire selon un axe orthogonal à l'axe formé par le rail 505. De plus ce module de piquée comporte un support libre en rotation comportant l'aiguille 510.

On observe sur la figure 6, schématiquement et en perspective, un quatrième mode de réalisation du dispositif 60 de ponction ou d'injection objet de la présente invention. Sur cette figure 6, on observe plus particulièrement un moyen de poussée de l'aiguille 605 pour que cette aiguille rentre et/ou sorte d'une veine par exemple.

On note que les dispositifs, 10, 20 et 40, tels que décrits en regard des figures, 1, 2 ou 4, peuvent être connectés, de manière filaire ou sans-fil, avec un terminal communicant, tel un ordinateur. Ce terminal communicant peut également être portable, tel un ordiphone ou une tablette numérique par exemple.

Ces dispositifs peuvent également communiquer avec une unité de calcul distante, tel un serveur par exemple. Les informations transmises à un tel serveur directement, ou indirectement par le biais d'un terminal communicant avec ledit dispositif, peuvent être mises en œuvre par une plateforme et/ou une application de suivi de patients.

Cette plateforme et/ou cette application met en œuvre, par exemple :
- des sessions d'utilisateurs pour les personnels de santé,
- une recherche d'utilisateur-patient,
- le déclenchement de la réalisation d'un traitement par saisie utilisateur,
- le suivi en temps réel d'un traitement en cours de réalisation, par affichage d'informations représentatives du traitement en cours sur un écran par exemple et/ou
- une visualisation d'une fiche d'un utilisateur-patient, cette fiche comportant des données relatives à la santé de cet utilisateur.

## Revendications

1. Dispositif (20) de ponction ou d'injection dans une veine d'un utilisateur, comprenant :
- un boitier opaque comportant une ouverture pour recevoir le bras de l'utilisateur,
- un moyen (215) de capture d'une image infrarouge du bras de l'utilisateur reçu dans le boitier,
- un moyen (220) de détection d'une veine dans l'image capturée,
- un moyen (225) de transmission d'une localisation de la veine détectée,
- une aiguille (230) de ponction ou d'injection,
le dispositif comprenant en outre :
- un dispositif (10) de maintien d'une veine d'un utilisateur en position, ledit dispositif de maintien comprenant :
∘ au moins deux branches séparées par un interstice d'une largeur supérieure à la dimension de la veine,
∘ un moyen (110) de positionnement desdites branches autour de la veine commandé en fonction de ladite localisation de la veine détectée,
∘ un moyen (130) de désinfection de la peau couvrant la veine de l'utilisateur,
- un moyen (235) de positionnement d'une extrémité de l'aiguille dans la veine détectée, entre les branches.

2. Dispositif (20) selon la revendication 1, qui comporte :
- un moyen (240) d'émission d'ultrason et,
- un moyen (245) de capture d'une image en fonction des ultrasons émis, le moyen (235) de positionnement positionnant l'aiguille en fonction de l'image captée.

3. Dispositif (20) selon l'une des revendications 1 ou 2, qui comporte un moyen (250) de permutation entre une aiguille (230) ayant réalisé une ponction et/ou une injection et une autre aiguille.

4. Dispositif (20) selon l'une des revendications 1 à 3, qui comporte un moyen (255) de maintien du bras de l'utilisateur dans ledit boîtier.

5. Dispositif (20) selon la revendication 4, dans lequel le moyen (255) de maintien comporte un garrot (260) se contractant automatiquement autour du bras de l'utilisateur et une poignée (265) pour recevoir la main fermée de l'utilisateur.

6. Dispositif (20) selon l'une des revendications 1 à 5, qui comporte :
- un moyen (270) d'aspiration de sang issu de la veine dans lequel l'aiguille (230) est positionnée et,
- un réservoir (275) amovible pour recevoir le sang aspiré.

7. Dispositif (20) selon la revendication 6, qui comporte :
- un moyen (280) d'accès à un profil de l'utilisateur,
- un moyen (285) d'identification du réservoir (275) avec une donnée du profil de l'utilisateur.

8. Dispositif selon l'une des revendications 1 à 7, dans lequel ledit dispositif (10) de maintien comporte :
- un moyen (115) d'échauffement d'au moins une branche (105) et,
- un moyen (120) de commande du moyen d'échauffement.

9. Dispositif selon l'une des revendications 1 à 8, dans lequel ledit dispositif (10) de maintien comporte un moyen (125) de refroidissement d'au moins une branche (105) et un moyen (120) de commande étant configuré pour actionner le moyen de refroidissement.

10. Dispositif selon l'une des revendications 1 à 9, dans lequel ledit moyen de positionnement (110) dudit dispositif de maintien (10) comporte un moyen (135) de pincement de la veine par les branches (105).

11. Dispositif selon l'une des revendications 1 à 10, dans lequel ledit dispositif de maintien (10) comporte un moyen d'émission de lumière ultraviolette en direction de la veine de l'utilisateur.

## Patentansprüche

1. Vorrichtung (20) zum Punktieren oder Injizieren in die Vene eines Benutzers, worin die Vorrichtung umfasst:
ein lichtundurchlässiges Gehäuse mit einer Öffnung zur Aufnahme des Arms des Benutzers,
ein Mittel (215) zum Erfassen eines Infrarotbildes des Armes des Benutzers in dem Gehäuse,
ein Mittel (220) zum Erkennen einer Vene in dem aufgenommenen Bild,
ein Mittel (225) zum Übertragen eines Ortes der erkannten Vene,
eine Punktions- oder Injektionsnadel (230),
worin die Vorrichtung ferner umfasst:
eine Einrichtung (10) zum Festhalten der Vene eines Benutzers in Position, worin die Einrichtung zum Festhalten der Vene umfasst:
mindestens zwei Schenkel, die durch einen Spalt getrennt sind, dessen Breite größer als die Abmessung der Vene ist,
ein Mittel (110) zum Anordnen der Schenkel um die kontrollierte Vene herum, entsprechend der Lage der erfassten Vene,
ein Mittel (130) zum Desinfizieren der Haut, die die Vene des Benutzers bedeckt,
ein Mittel (235) zum Positionieren eines Endes der Nadel in der erfassten Vene zwischen den Schenkeln.

2. Vorrichtung (20) nach Anspruch 1, welche umfasst:
ein Mittel (240) zum Aussenden von Ultraschall und,
ein Mittel (245) zum Erfassen eines Bildes in Abhängigkeit von dem übertragenen Ultraschall, wobei das Anordnungsmittel (235) die Nadel in Abhängigkeit von dem erfassten Bild anordnet.

3. Vorrichtung (20) nach einem der Ansprüche 1 oder 2, welche ein Mittel (250) zum Umschalten zwischen einer Nadel (230), die eine Punktion und/oder eine Injektion durchgeführt hat, und einer anderen Nadel umfasst.

4. Vorrichtung (20) nach Anspruch 1 bis 3, welche ein Mittel (255) zum Halten des Armes des Benutzers in dem Gehäuse umfasst.

5. Vorrichtung (20) nach Anspruch 4, worin das Haltemittel (255) eine Druckbinde (260) umfasst, die sich automatisch um den Arm des Benutzers zusammenzieht, und einen Griff (265) zur Aufnahme der geschlossenen Hand des Benutzers umfasst.

6. Vorrichtung (20) nach einem der Ansprüche 1 bis 5, welche umfasst:
ein Mittel (270) zur Blutentnahme aus der Vene, in der die Nadel (230) positioniert ist, und,
ein abnehmbares Reservoir (275) zur Aufnahme des angesaugten Blutes.

7. Vorrichtung (20) nach Anspruch 6, welche umfasst:
ein Mittel (280) für den Zugriff auf ein Benutzerprofil,
ein Mittel (285) zur Identifizierung des Reservoirs (275) mit Daten eines Benutzerprofils.

8. Vorrichtung nach Anspruch 1 bis 7, worin die Haltevorrichtung (10) umfasst:
eine Einrichtung (115) zum Beheizen mindestens eines Schenkels (105) und,
ein Mittel (120) zur Steuerung der Heizmittel.

9. Vorrichtung nach einem der Ansprüche 1 bis 8, worin die Haltevorrichtung (10) ein Mittel (125) zum Kühlen mindestens eines Schenkels (105) und ein Steuermittel (120) umfasst, das ausgestaltet ist, das Kühlmittel zu betreiben.

10. Vorrichtung nach einem der Ansprüche 1 bis 9, worin das Anordnungsmittel (110) der Haltevorrichtung (10) ein Mittel (135) zum Abklemmen der Vene durch die Schenkel (105) umfasst.

11. Vorrichtung nach einem der Ansprüche 1 bis 10, worin die Haltevorrichtung (10) ein Mittel zum Aussenden von ultraviolettem Licht in Richtung der Vene des Anwenders umfasst.

## Claims

1. Device (20) for puncturing or injection into a user's vein comprising:
- an opaque casing (205) comprising an opening (210) to receive the user's arm.
- a means (215) for capturing an infrared image of the user's arm lodged in the casing,
- a means (220) for detecting a vein in the captured image,
- a means (225) for transmitting a location of the vein detected,
- a needle (230) for puncturing or injection,
the device further comprising :
- a device (10) for maintaining a user's vein in position, such device comprising :
∘ at least two branches separated by a gap of with greater than the size of the vein,
∘ a means (110) for positioning said branches around the user's vein in function of the location of the vein detected,
∘ a means (130) for disinfecting the skin covering the user's vein.
∘
- a means (235) for positioning an extremity of the needle in the vein detected,

2. Device (20) according to claim 1, which comprises:
- a means (240) for emitting ultrasound; and
- a means (245) for capturing an image as a function of the ultrasounds emitted, the positioning means (235) positioning the needle as a function of the image captured.

3. Device (20) according to one of claims 1 or 2, which comprises a means (250) for switching between a needle (230) that has performed a puncture and/or an injection and another needle.

4. Device (20) according to one of claims 1 to 3, which comprises a means (255) for maintaining the arm of the user in the casing.

5. Device (20) according to claim 4, in which maintenance means (255) comprises a tourniquet (260) contracting automatically around the user's arm and a handle (265) to receive the user's closed hand.

6. Device (20) according to one of claims 1 to 5, which comprises:
- a means (270) for aspirating blood from the vein in which the needle (230) is positioned; and
- a removable reservoir (275) for receiving the blood aspirated.

7. Device (20) according to claim 6, which comprises:
- a means (280) for accessing a profile of the user;
- a means (285) for identifying the reservoir (275) with an item of data of the user's profile.

8. Device (20) according to any one of claims 1 to 7, which comprises:
- a means (115) of heating at least one branch (105), and,
- a means (120) for controlling the heating means.

9. Device (10) according to any one claims 1 to 8, which comprises a means (125) for cooling at least one branch (105) and a control means (120) configured to activate the cooling means.

10. Device (10) according to one of claims 1 to 9, wherein the positioning means (110) comprises a means (135) for pinching the vein by the branches (105).

11. Device (10) according to any one of claims 1 to 10, which comprises a means (125) for emitting ultraviolet light in the direction of the user's vein.
